# EUROPEAN PATENT SPECIFICATION

(11) **EP 4 011 344 B1**
(45) Date of publication and mention of the grant of the patent: **11.09.2024**
(21) Application number: 21153723.8
(22) Date of filing: 27.01.2021
(51) Int. Cl.: A61F 13/531, A61F 13/515, A61F 13/532, C09J 7/00, B32B 7/14, C09J 9/00, C09J 11/00, C09J 113/00

(54) **ABSORBENT ARTICLES**
ABSORBIERENDE ARTIKEL
ARTICLES ABSORBANTS

(30) Priority: 10.12.2020 EP 20213184
(43) Date of publication of application: 15.06.2022
(73) Proprietor: Ontex BV, 9255 Buggenhout (BE); Ontex Group NV, 9320 Erembodegem (BE)
(72) Inventor: Granado, Martín, 56727 Mayen (DE); Ingenfeld, Björn, 53115 Bonn (DE); Saheb Mohammed, Moula, 56727 Mayen (DE); Lampe, Ulrich, 56727 Mayen (DE); Lambertz, Christina, 56566 Neuwied (DE)
(74) Representative: Larangé, Françoise

(56) References cited:
- EP-A1- 2 949 299
- EP-A1- 3 473 222
- US-A1- 2018 256 416
- US-A1- 2020 375 811

## Description

### TECHNICAL FIELD

The present disclosure pertains to the technical field of absorbent hygiene products and relates to an absorbent core that can be used within an article for absorbing body fluids and exudates, such as urine and fecal material, or blood, menses, and vaginal fluids. In particular, the present disclosure relates to absorbent garments (or articles), such as disposable diapers or diaper pants, disposable incontinence diapers or pants, which are configured to collect and contain fecal material and avoid leakage, or sanitary napkins or panty liners, which are configured to collect and contain blood, menses, urine, vaginal fluids and avoid leakage. More particularly the present disclosure relates to absorbent cores having one or more channels therethrough.

### BACKGROUND

Absorbent cores have been subject to considerable improvement and innovation over time to address needs such as improved fluid absorption and distribution, as well as comfort, and a need for continued improvement exists. Such needs are ever present in today's demanding consumer environment.

In this context, more recently, absorbent cores having one or more channels substantially free of absorbent material have been developed.

EP3342386 discloses an absorbent core comprising substantially continuous zones of one or more high fluid distribution structures and discontinuous zones of fluid absorption structures surrounding the one or more high fluid distribution structures, wherein the one or more high fluid distribution structures are arranged to distribute fluid across the absorbent core at a speed that is faster than the speed of fluid distribution across the absorbent core by said discontinuous fluid absorption structures, and wherein said continuous zones extend along a path that is substantially parallel to at least a portion of the perimeter of the core, said portion of the perimeter of the core comprising at least a portion of the sides of the core and one of the ends of the core. In particular it describes one or more high fluid distribution structures consisting of two nonwoven webs bonded together.

WO2019/158226 discloses an absorbent core particularly designed to improve uniform liquid distribution and comfort, comprising at least one interconnected channel substantially free of absorbent material, formed by mechanically bonding said upper layer directly to said lower layer at a plurality of distinct bonding sites.

Although channels are beneficial in terms of fluid handling, there still remains a need to further improve liquid distribution, whilst ensuring comfort and fit, in dry and wet state.

EP2949299 discloses an absorbent core which may comprise a pair of channel-forming areas. It describes that a glue, when present, may at least partially help forming the core wrap bond within the channel-forming areas. Said glue may comprise or consist of any kind of thermoplastic hot-melt adhesives used in the field of absorbent core making.

US2018256416 discloses an absorbent body comprising fibers and high absorbent polymer particles enclosed in a wrapping sheet where distal ends of the both side parts of the wrapping sheet are overlapped on the back face side of the absorbent body to form a connecting portion. A slit may be present, penetrating the absorbent body in a thickness direction and extending in the front-back direction at an intermediate portion of the absorbent body in the width direction.

EP3473222 discloses absorbent articles with different types of channels. It describes a bond which provides for structural integrity of the channels in dry and wet state, and which can result from any known bonding techniques known in the art, in particular one selected from adhesive bonding, thermobonding, mechanical bonding, ultrasonic bonding, or any combinations thereof.

US2020375811 discloses ultrathin absorbent hygienic pads comprising a top layer, a middle absorbent layer, and a bottom layer and comprising one or more channels anchored from the top layer through the middle absorbent layer and into the bottom layer.

The present disclosure aims to provide a novel absorbent article utilizing a channeled core particularly designed to provide exceptionally strong bonding and excellent stability within the channel(s), even in wet state. It offers good liquid distribution throughout the entire core during entire use of the article, leading to improved absorption capacity. It further offers an improved fit to the wearer in dry and wet state, thereby providing comfort and minimizing the risk of leakage.

### SUMMARY

In one aspect, the present disclosure relates to an absorbent article comprising a liquid permeable topsheet, a liquid impermeable backsheet and an absorbent core positioned between said topsheet and said backsheet. The absorbent core comprises absorbent material selected from the group consisting of cellulose fibers, superabsorbent polymers and combinations thereof and the absorbent material is contained within at least one core wrap substrate enclosing said absorbent material. A top layer of said core wrap is bonded via a hot melt adhesive to a bottom layer of said core wrap at one or more attachment zones to form one or more channels substantially free of absorbent material. The hot melt adhesive is selected from the group consisting of rubber-based adhesives, adhesives having a viscosity of at least 5500 mPa·s at 150°C and rubber-based adhesives having a viscosity of at least 5500 mPa·s at 150°C.

We have found that having an adhesive of the rubber-based type and/or having a viscosity of at least 5500 mPa·s at 150°C, to attach top and bottom core wrap layers together within the channel(s) improves the stability of the channel(s) especially when getting wet.

Stability of the channel(s) is important over time, to maintain the liquid distribution properties and the comfort and fit that the channel(s) are offering, throughout the duration of use of the article on a wearer.

Other objects and advantages of this invention will become apparent hereinafter.

### BRIEF DESCRIPTION OF THE FIGURES

Fig. 1 A-D illustrates absorbent articles according to embodiments according to the present disclosure.
Fig. 2 to 5 illustrate the Hang-Peel Test as herein described.
Fig. 6 shows a graph comparing viscosity values in mPa·s (Axis Y) of various adhesives according to temperature in °C (Axis X).

### DETAILED DESCRIPTION

Unless otherwise defined, all terms used in disclosing characteristics of the disclosure, including technical and scientific terms, have the meaning as commonly understood by one of ordinary skill in the art to which this disclosure belongs. By means of further guidance, term definitions are included to better appreciate the teaching of the present disclosure.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1 % or less, and still more preferably +/-0.1 % or less of and from the specified value, in so far such variations are appropriate to perform in the disclosed disclosure. However, it is to be understood that the value to which the modifier "about" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The expression "% by weight" (weight percent), here and throughout the description unless otherwise defined, refers to the relative weight of the respective component based on the overall weight of the formulation.

The recitation of numerical ranges by endpoints includes all numbers and fractions subsumed within that range, as well as the recited endpoints.

"Acquisition and distribution layer", "ADL", "Acquisition and distribution system" or "surge management portion" refers to a sub-layer which preferably is a nonwoven wicking layer under the top sheet of an absorbent product, which speeds up the transport and improves distribution of fluids throughout the absorbent core. The surge management portion is typically less hydrophilic than the retention portion, and has the ability to quickly collect and temporarily hold liquid surges, and to transport the liquid from its initial entrance point to other parts of the absorbent structure, particularly the retention portion. This configuration can help prevent the liquid from pooling and collecting on the portion of the absorbent garment positioned against the wearer's skin, thereby reducing the feeling of wetness by the wearer. Preferably, the surge management portion is positioned between the top sheet and the retention portion.

The term "adhesive" as used herein is intended to refer to any suitable hot melt, water or solvent borne adhesive that can be applied to a surface of a product component of the present disclosure. Accordingly, suitable adhesives include conventional hot melt adhesives, pressure-sensitive adhesives and reactive adhesives (i.e., polyurethane). "Hot melt adhesive" as used herein refers to adhesives applied from the melt and gaining strength upon solidification.

As used herein, the term "adhesive bonding" means a bonding process which forms a bond by application of an adhesive. Such application of adhesive may be by various processes such as slot coating, spray coating and other topical applications. Further, such adhesive may be applied within a product component and then exposed to pressure such that contact of a second product component with the adhesive containing product component forms an adhesive bond between the two components.

The term "back sheet" or "backsheet" refers to a material forming the outer cover of the absorbent article. The back sheet prevents the exudates contained in the absorbent structure from wetting articles such as bedsheets and overgarments which contact the disposable absorbent article. The back sheet may be a unitary layer of material or may be a composite layer composed of multiple components assembled side-by-side or laminated. The back sheet may be the same or different in different parts of the absorbent article. At least in the area of the absorbent medium the back sheet comprises a liquid impervious material in the form of a thin plastic film, e.g. a polyethylene or polypropylene film, a nonwoven material coated with a liquid impervious material, a hydrophobic nonwoven material, which resists liquid penetration, or a laminate of a plastic film and a nonwoven material. The back sheet material may be breathable so as to allow vapour to escape from the absorbent material, while still preventing liquids from passing there through. Examples of breathable back sheet materials are porous polymeric films, nonwoven laminates of spunbond and meltblown layers and laminates of porous polymeric films and nonwoven materials.

As used herein, the "skin facing", "body-facing" or "bodyside" surface means that surface of the article or component which is intended to be disposed toward or placed adjacent to the body of the wearer during ordinary use, while the "outward", "outward-facing" or "garment-side" or "garment facing" surface is on the opposite side, and is intended to be disposed to face away from the wearer's body during ordinary use. Such outward surface may be arranged to face toward or placed adjacent to the wearer's undergarments when the absorbent article is worn.

"Bonded" refers to the joining, adhering, connecting, attaching, or the like, of at least two elements. Two elements will be considered to be bonded together when they are bonded directly to one another or indirectly to one another, such as when each is directly bonded to intermediate elements.

"Carded web (or layer(s) or nonwoven)" refers to webs that are made from staple fibers that are sent through a combing or carding unit, which opens and aligns the staple fibers in the machine direction to form a generally machine direction-oriented fibrous nonwoven web. The web is then bonded by one or more of several known bonding methods. Bonding of nonwoven webs may be achieved by a number of methods; powder bonding, wherein a powdered adhesive or a binder is distributed through the web and then activated, usually by heating the web and adhesive with hot air; pattern bonding, wherein heated calendar rolls or ultrasonic bonding equipment are used to bond the fibers together, usually in a localized bond pattern, though the web can be bonded across its entire surface if so desired; through-air bonding, wherein air which is sufficiently hot to soften at least one component of the web is directed through the web; chemical bonding using, for example, latex adhesives that are deposited onto the web by, for example, spraying; and consolidation by mechanical methods such as needling and hydroentanglement. Carded thermobonded nonwoven thus refers to a carded nonwoven wherein the bonding is achieved by use of heat.

As used herein, the term "cellulosic" or "cellulose" is meant to include any material having cellulose as a major constituent, and specifically comprising at least 50 percent by weight cellulose or a cellulose derivative. Thus, the term includes cotton, typical wood pulps, nonwoody cellulosic fibers, cellulose acetate, cellulose triacetate, rayon, thermomechanical wood pulp, chemical wood pulp, debonded chemical wood pulp, milkweed, or bacterial cellulose.

The term "consisting essentially of" does not exclude the presence of additional materials which do not significantly affect the desired characteristics of a given composition or product. Exemplary materials of this sort would include, without limitation, pigments, antioxidants, stabilizers, surfactants, waxes, flow promoters, solvents, particulates and materials added to enhance processability of the composition.

"Hot melt adhesive" means a formulation that generally comprises several components. These components typically include one or more polymers to provide cohesive strength (e.g., aliphatic polyolefins such as poly (ethylene-co-propylene) copolymer; ethylene vinyl acetate copolymers; styrene-butadiene or styrene- isoprene block copolymers; etc.); a resin or analogous material (sometimes called a tackifier) to provide adhesive strength (e.g., hydrocarbons distilled from petroleum distillates; rosins and/or rosin esters; terpenes derived, for example, from wood or citrus, etc.); perhaps waxes, plasticizers or other materials to modify viscosity (i.e., flowability) (examples of such materials include, but are not limited to, mineral oil, polybutene, paraffin oils, ester oils, and the like); and/or other additives including, but not limited to, antioxidants or other stabilizers. A typical hot-melt adhesive formulation might contain from about 15 to about 35 weight percent cohesive strength polymer or polymers; from about 50 to about 65 weight percent resin or other tackifier or tackifiers; from more than zero to about 30 weight percent plasticizer or other viscosity modifier; and optionally less than about 1 weight percent stabilizer or other additive. It should be understood that other adhesive formulations comprising different weight percentages of these components are possible.

The term "disposable" is used herein to describe absorbent articles that generally are not intended to be laundered or otherwise restored or reused as an absorbent article (i.e., they are intended to be discarded after a single use and, preferably, to be recycled, composted or otherwise disposed of in an environmentally compatible manner).

"Join", "joining", "joined", or variations thereof, when used in describing the relationship between two or more elements, means that the elements can be connected together in any suitable manner, such as by heat sealing, ultrasonic bonding, thermal bonding, by adhesives, stitching, or the like. Further, the elements can be joined directly together, or may have one or more elements interposed between them, all of which are connected together.

"Laminate" refers to elements being attached together in a layered arrangement.

The use of the term "layer" can refer, but is not limited, to any type of substrate, such as a woven web, nonwoven web, films, laminates, composites, elastomeric materials, or the like. A layer can be liquid and air permeable, permeable to air but impermeable to liquids, impermeable both to air and liquid, or the like. When used in the singular, it can have the dual meaning of a single element or a plurality of elements, such as a laminate or stacked plural sub-layers forming a common layer.

"Liquid" means a nongaseous substance and/or material that flows and can assume the interior shape of a container into which it is poured or placed.

"Longitudinal" is a direction running parallel to the maximum linear dimension of the article.

By the terms "particle", "particles", "particulate", "particulates" and the like, it is meant that the material is generally in the form of discrete units. The units can comprise granules, powders, spheres, pulverized materials or the like, as well as combinations thereof. The particles can have any desired shape such as, for example, cubic, rod-like, polyhedral, spherical or semi-spherical, rounded or semi-rounded, angular, irregular, etc. Shapes having a large greatest dimension/smallest dimension ratio, like needles, flakes and fibers, are also contemplated for inclusion herein. The terms "particle" or "particulate" may also include an agglomeration comprising more than one individual particle, particulate or the like. Additionally, a particle, particulate or any desired agglomeration thereof may be composed of more than one type of material.

The term "polymer" generally includes, but is not limited to, homopolymers, copolymers, such as, for example, block, graft, random and alternating copolymers, terpolymers, etc. and blends and modifications thereof. Furthermore, unless otherwise specifically limited, the term "polymer" shall include all possible geometrical configurations of the material. These configurations include, but are not limited to, isotactic, syndiotactic and random symmetries.

"Pulp fluff" or "fluff pulp" refers to a material made up of cellulose fibers. The fibers can be either natural or synthetic, or a combination thereof. The material is typically lightweight and has absorbent properties.

The "retention portion" or "liquid absorption layer" is part of the absorbent medium. This portion may comprise a matrix of hydrophilic fibers, such as a web of cellulosic fluff, mixed with particles of high absorbency material. In particular arrangements, the retention portion may comprise a mixture of superabsorbent hydrogel-forming particles and synthetic polymer meltblown fibers, or a mixture of superabsorbent particles with a fibrous coform material comprising a blend of natural fibers and/or synthetic polymer fibers. The superabsorbent particles may be substantially homogeneously mixed with the hydrophilic fibers, or may be nonuniformly mixed. For example, the concentrations of superabsorbent particles may be arranged in a non-step-wise gradient through a substantial portion of the thickness of the absorbent structure, with lower concentrations toward the bodyside of the absorbent structure and relatively higher concentrations toward the outerside of the absorbent structure. The superabsorbent particles may also be arranged in a generally discrete layer within the matrix of hydrophilic fibers. In addition, two or more different types of superabsorbent may be selectively positioned at different locations within or along the fiber matrix.

As used herein the term "sheet" or "sheet material" refers to woven materials, nonwoven webs, polymeric films, polymeric scrim-like materials, and polymeric foam sheeting.

The term "spunbond fibers (or layer(s) or nonwovens)" refers to fibers formed by extruding molten thermoplastic polymers as filaments or fibers from a plurality of relatively fine, usually circular, capillaries of a spinneret, and then rapidly drawing the extruded filaments by an eductive or other well-known drawing mechanism to impart molecular orientation and physical strength to the filaments. The average diameter of spunbond fibers is typically in the range of from 15-60 µm or higher. The spinneret can either be a large spinneret having several thousand holes per meter of width or be banks of smaller spinnerets, for example, containing as few as 40 holes.

The term "spunbond meltblown spunbond" (SMS) nonwoven fabric as used herein refers to a multi-layer composite sheet comprising a web of meltblown fibers sandwiched between and bonded to two spunbond layers. A SMS nonwoven fabric can be formed in-line by sequentially depositing a first layer of spunbond fibers, a layer of meltblown fibers, and a second layer of spunbond fibers on a moving porous collecting surface. The assembled layers can be bonded by passing them through a nip formed between two rolls that can be heated or unheated and smooth or patterned. Alternately, the individual spunbond and meltblown layers can be pre-formed and optionally bonded and collected individually such as by winding the fabrics on wind-up rolls. The individual layers can be assembled by layering at a later time and bonded together to form a SMS nonwoven fabric. Additional spunbond and/or meltblown layers can be incorporated to form laminate layers, for example spunbond-meltblown-meltblown-spunbond (SMMS), or spunbond-meltblown (SM) etc.

"Staple fibers" refer to commercially available fibers having diameters ranging from less than about 0.001 mm to more than about 0.2 mm; they come in several different forms such as short fibers ranging from about 10 to 50 mm in length and long fibers with a length higher than 50 mm, preferably up to 100 mm.

"Spunlaced" as used herein refers to nonwoven fabrics or materials that are made by hydroentangling webs of fibers (and/or fibers) with high energy water jets for example as basically described in Evans et al. US Patent No. 3,485,706. The webs may be made of a variety of fibers such as polyester, rayon, cellulose (cotton and wood pulp), acrylic, and other fibers as well as some blends of fibers. The fabrics may be further modified to include antistatic and antimicrobial properties, etc. by incorporation of appropriate additive materials into the fiber or fiber webs.

"Wetlaid" as used herein means nonwovens obtained by a process similar to paper manufacturing. The difference lies in the amount of synthetic fibres present in a wetlaid nonwoven. A dilute slurry of water and fibres is deposited on a moving wire screen, where the water is drained and the fibres form a web. The web is further dewatered by pressing between rollers and dried. Impregnation with binders is often included in a later stage of the process.

"Airlaid" as used herein means a process wherein fibres, which are typcially relatively short, are fed into a forming head by an airstream. The forming head assures a homogeneous mix of all fibres. By air again, a controlled part of the fibre mix leaves the forming head and is deposited on a moving belt, where a randomly oriented web is formed. Compared with carded webs, airlaid webs have a lower density, a greater softness and an absence of laminar structure.

As used herein "channels" are fluid distribution means within the absorbent core adapted to favour exudate flow there along and are typically intended to exclude embossing patterns or ducts formed by compression from the meaning thereof and rather include structures that are substantially free of absorbent material instead of comprising compacted absorbent material. Channels herein are formed by joining upper and lower layers of a core wrap as will be described in more detail herein below. Channels preferably comprise recessed regions forming visible conduits or passages typically extending along the longitudinal axis of the core and having a depth in a direction perpendicular to said longitudinal axis. By "visible" it is herein intended clearly visible by naked eye. Typically the channels have a width generally greater than 1mm, preferably from 5mm to 50mm, more preferably from 6mm to 40mm, more preferably from 8mm to 30mm, even more preferably from greater than 8mm to less than 25mm.

By "substantially", it is meant at least the majority of the structure referred to. For example, with reference to a channel following "a substantially continuous path from any point of said channel to any other point of the same channel", this means that the majority of the channel is interconnected and generally wherein a direct and continuous path can be traced by starting from one point of the channel towards another point of the channel.

By the term "substantially U-shaped" as used herein, is meant any shape that visually approximates the shape of a "U", such as a "V-shape", a semi-circle, and the like.

By "directly over", it is meant that the feature referred to is placed over the structure referred to such that the two are in direct contact with each other at least throughout a substantial portion of said structure.

By "indirectly over", it is meant that the feature referred to is placed over the structure referred to but in such a way that the two are not in direct contact with each other at least throughout a substantial portion of said structure. For example, a nonwoven web applied indirectly over a three-dimensional absorbent material comprises a further layer of material between said nonwoven web and said three-dimensional absorbent material.

Use of the term "substrate" includes, but is not limited to, woven or nonwoven webs, porous films, ink permeable films, paper, composite structures, or the like.

The terms "Superabsorbent" or "high absorbency" refer to materials that are capable of absorbing at least 10 times their own weight in liquid. Superabsorbent materials suitable for use in the present disclosure are known to those skilled in the art, and may be in any operative form, such as particulate form, fibers and mixtures thereof. Generally stated, the "superabsorbent material" can be a water-swellable, organic or inorganic, generally water-insoluble, hydrogel-forming polymeric absorbent material, which is capable of absorbing at least about 10 or 15, suitably about 30, and possibly about 60 times or more its weight in physiological saline (e.g. saline with 0.9 wt % NaCl). The superabsorbent material may be biodegradable or bipolar. The hydrogel-forming polymeric absorbent material may be formed from organic hydrogel-forming polymeric material, which may include natural material such as agar, pectin, and guar gum; modified natural materials such as carboxymethyl cellulose, carboxyethyl cellulose, and hydroxypropyl cellulose; and synthetic hydrogel-forming polymers. Synthetic hydrogel-forming polymers include, for example, alkali metal and ammonium salts of polyacrylic acid and polymethacrylic acid, polyacrylamides, polyvinyl alcohol, ethylene maleic anhydride copolymers, polyvinyl ethers, polyvinyl morpholinone, polymers and copolymers of vinyl sulfonic acid, polyacrylates, polyacrylamides, polyvinyl pyridine, maleic anhydride copolymers with vinyl ethers and alpha-olefins, poly(vinyl pyrrolidone), poly(vinylmorpholinone), poly(vinyl alcohol), and the like, and mixtures and copolymers thereof. Other suitable hydrogel-forming polymers include hydrolyzed acrylonitrile grafted starch, acrylic acid grafted starch, and isobutylene maleic anhydride copolymers and mixtures thereof, methyl cellulose, carboxymethyl cellulose, hydroxypropyl cellulose, and the natural gums, such as alginates, xanthan gum, locust bean gum and the like. The hydrogel-forming polymers may be inorganic materials, such as silica gels, or organic compounds such as cross-linked polymers. The term "cross-linked" refers to any means for effectively rendering normally water-soluble materials substantially water insoluble but swellable. Such means can include, for example, physical entanglement, irradiation, crystalline domains, covalent bonds, ionic complexes and associations, hydrophilic associations, such as hydrogen bonding, and hydrophobic associations or Van der Waals forces. Mixtures of natural and wholly or partially synthetic absorbent polymers can also be used. Synthetic hydrogel-forming materials typically are xerogels which form hydrogels when wetted. The term "hydrogel", however, has commonly been used to also refer to both the wetted and unwetted forms of the material. The superabsorbent material may be in any of a wide variety of geometric forms. As a general rule, it is preferred that the superabsorbent material be in the form of discrete particles. However, the superabsorbent material may also be in the form of fibres, flakes, rods, spheres, needles, spiral or semi-spiral, cubic, rod-like, polyhedral, or the like. Conglomerates of particles of superabsorbent material may also be used. The superabsorbent material may suitably be included in an appointed storage or retention portion of the absorbent system, and may optionally be employed in other components or portions of the absorbent article. The superabsorbent material may be included in the absorbent layer or other fluid storage layer of the absorbent article in an amount of from about 5 to about 100 weight percent and desirably from about 30 to about 100 weight percent based on the total weight of the absorbent core. The distribution of the high-absorbency material within the different portions of the absorbent core can vary depending upon the intended end use of the absorbent core. The high-absorbency material may be arranged in a generally discrete layer within the matrix of hydrophilic fibres. Alternatively, the absorbent core may comprise a laminate of fibrous webs and high-absorbency material or other suitable means of maintaining a high-absorbency material in a localized area.

"Superabsorbent polymer particles" or "SAPs" refer to water-swellable, water-insoluble organic or inorganic materials capable, under the most favorable conditions, of absorbing at least about 10 times their weight, or at least about 15 times their weight, or at least about 25 times their weight in an aqueous solution containing 0.9 weight percent sodium chloride. In absorbent articles, such as diapers, incontinent diapers, etc., the particle size is typically ranging between 100 to 800 µm, preferably between 300 to 600 µm, more preferably between 400 to 500 µm.

As used herein, the term "thermoplastic" is meant to describe a material that softens when exposed to heat and which substantially returns to its original condition when cooled to room temperature.

The term "top sheet" or "topsheet" refers to a liquid permeable material sheet forming the inner cover of the absorbent article and which in use is placed in direct contact with the skin of the wearer. The top sheet is typically employed to help isolate the wearer's skin from liquids held in the absorbent structure. The top sheet can comprise a nonwoven material, e.g. spunbond, meltblown, carded, hydroentangled, wetlaid etc. Suitable nonwoven materials can be composed of man-made fibres, such as polyester, polyethylene, polypropylene, viscose, rayon etc. or natural fibers, such as wood pulp or cotton fibres, or from a mixture of natural and man-made fibres. The top sheet material may further be composed of two fibres, which may be bonded to each other in a bonding pattern. Further examples of top sheet materials are porous foams, apertured plastic films, laminates of nonwoven materials and apertured plastic films etc. The materials suited as top sheet materials should be soft and non-irritating to the skin and be readily penetrated by body fluid, e.g. urine or menstrual fluid. The inner coversheet may further be different in different parts of the absorbent article. The top sheet fabrics may be composed of a substantially hydrophobic material, and the hydrophobic material may optionally be treated with a surfactant or otherwise processed to impart a desired level of wettability and hydrophilicity. As used herein, the term "transverse" or "lateral" refers to a line, axis, or direction which lies within the plane of the absorbent article and is generally perpendicular to the longitudinal direction.

"Dry state" refers to the condition in which an absorbent article has not yet been saturated with exudates and/or liquid.

"Wet state" refers to the condition in which an absorbent article has been saturated with exudates and/or liquid. Typically wherein at least 30ml, preferably at least 40ml, even more preferably at least 50ml, most preferably from 60ml to 800ml, of exudate and/or liquid are contained in the absorbent article.

By the term "superabsorbent polymer fibers" as used herein, is meant fibers made from superabsorbent polymers (as opposed to particles made therefrom). Examples of suitable fibers for use herein are selected from those of Example 1, Example 2, Example 3, and/or Example 4 (page 5, lines 1-46) of EP3190216A1 Saic fibers typically being used to form nonwoven webs or substrates according to the same referenced application.

By the terms "longitudinally-, transversally-, diagonally- -extending" as used herein, is meant a general orientation substantially parallel respectively to the longitudinal axis, to the transversal axis, and to any axis between them. This covers deviations from the axis used as reference of between 0° and 20°. This may cover straight lines but also curved lines. In the latter case, it is the main general orientation of the curve which is meant to be described.

By the term "center line" as used herein, is meant an axis dividing the element to which it refers, in two portions of equal length on either side of this axis.

Embodiments of the articles and processes according to the disclosure will now be described. It is understood that technical features described in one or more embodiments maybe combined with one or more other embodiments without departing from the intention of the disclosure and without generalization therefrom.

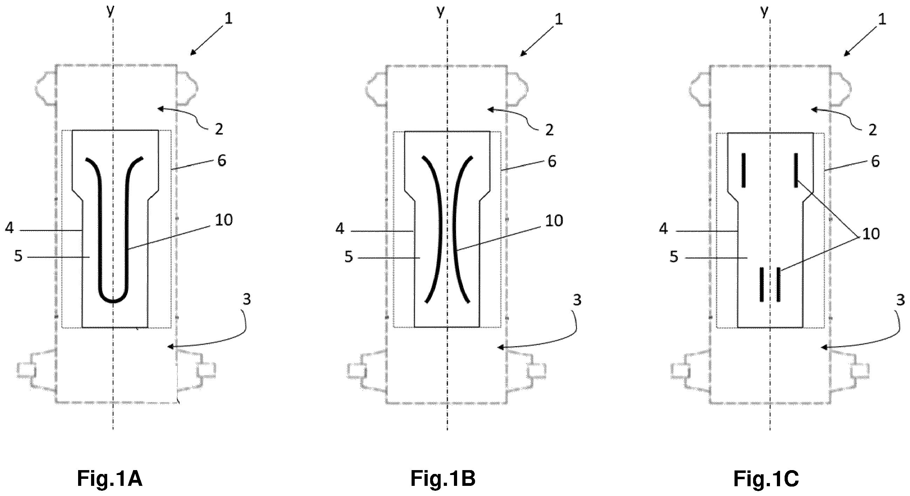

As exemplified in Fig. 1A-D, absorbent articles herein comprise a liquid permeable topsheet (2), a liquid impermeable backsheet (3) and an absorbent core (4) positioned between said topsheet (2) and said backsheet (3), wherein the absorbent core (4) comprises an absorbent material (5) selected from the group consisting of cellulose fibers, superabsorbent polymers and combinations thereof, and wherein said absorbent material (5) is contained within at least one core wrap substrate (6) enclosing said absorbent material (5) therein. The core wrap substrate (6) may be a single sheet that is folded onto itself to contain the absorbent material therein or may comprise two distinct sheets that are bonded to each other at least at the peripheral edges thereof to sandwich the absorbent material therein. Several core wrap constructions are possible such as G-fold, C-fold or sandwich wraps. The core wrap substrates herein are typically composed of a nonwoven layer or nonwoven laminates comprising spunbond nonwoven, meltblown nonwoven and combinations thereof or tissue. Typically the basis weight of the core wrap substrate is from 5 gsm to 50 gsm, preferably from 6 gsm to 35 gsm, more preferably from 7 gsm to 30 gsm.

The core wrap substrate (6) comprises an upper or top layer and a lower or bottom layer and the absorbent material is sandwiched therebetween with the top layer positioned at a skin-facing (or body-facing) side of the absorbent material and the bottom layer positioned at an opposite garment facing side of said absorbent material.

The top and bottom layers are joined together at one or more attachment zones positioned inboard of a perimeter of the absorbent core such to form one or more channels (10) substantially free of absorbent material. The channel(s) typically have a length extending in the longest dimension of the core that is from 10% to 95%, preferably from 15% to 90%, even more preferably from 20% to 85%, of the length of said longest dimension of the core. Exemplary channel geometries are illustrated in Fig. 1A-D. The top and bottom layers of the core wrap are joined together at the attachment zones via a hot melt adhesive selected from the group consisting of rubber-based adhesives, adhesives having a viscosity of at least 5500 mPa·s at 150°C and rubber-based adhesives having a viscosity of at least 5500 mPa·s at 150°C.

According to the invention, the one or more channels are formed by the adhesion of the top layer of the core wrap with the bottom layer of said core wrap, in zones substantially free of absorbent material. Such adhesion advantageously provides core stability and for example reduces the risk of sagging.

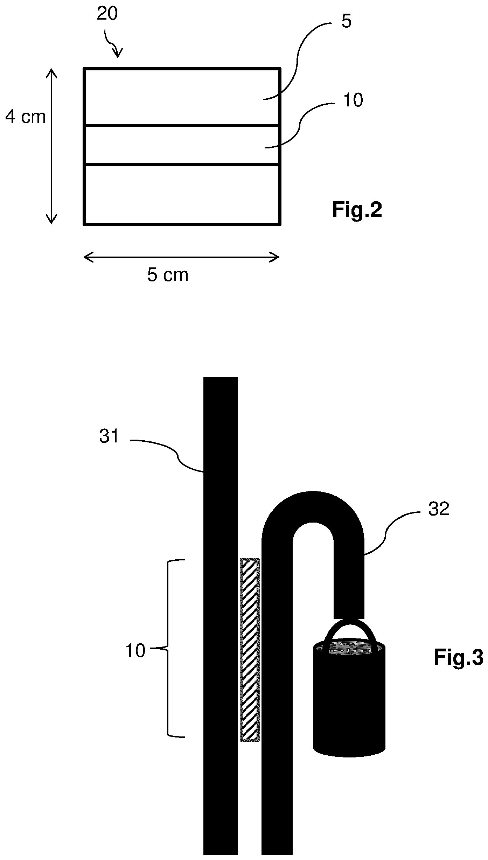

In order to identify which features of an adhesive are important to ensure channel stability even in wet state, it has been necessary to first find an appropriate test, matching the reality, to measure the adhesion force within the channel. We have found for example that well-known ZWICK shear or peel tests, in dynamic mode, were not appropriate. Similarly, static hang shear tests were also found unsuitable. Other tests, wherein an absorbent article is soaked in a saline solution and spun in a centrifuge and wherein the channel stability is thereafter visually controlled, turned out unreliable due to subjective judgement. We have therefore developed a new method of assessing the channel stability when wet, on a dry sample. This test is called: Hang Peel Test at 38°C and is explained hereinbelow. When SAP is swelling in absorbent articles being used, a force is acting on the glued channel attachment zones. In order to have a quantitative test method assessing the channel bonding performance, we have developed the Hang Peel Test performed at 38°C mimicking body temperature. In this test, the force acting by a swollen SAP on the channel area is translated into a peel configuration for the top and bottom core wrap layers glued together in the channel area (see Fig.3). This method has the advantage of being able to be carried out on a dry article, and still be reliable to quantify the channel bonding performance of a wet article in use.

We have further found that adhesives performing better in the Hang Peel Test at 38°C, and therefore offering better channel stability in dry and wet state, were rubber-based adhesives or adhesives having a viscosity of at least 5500 mPa·s at 150°C, and adhesives having both these features.

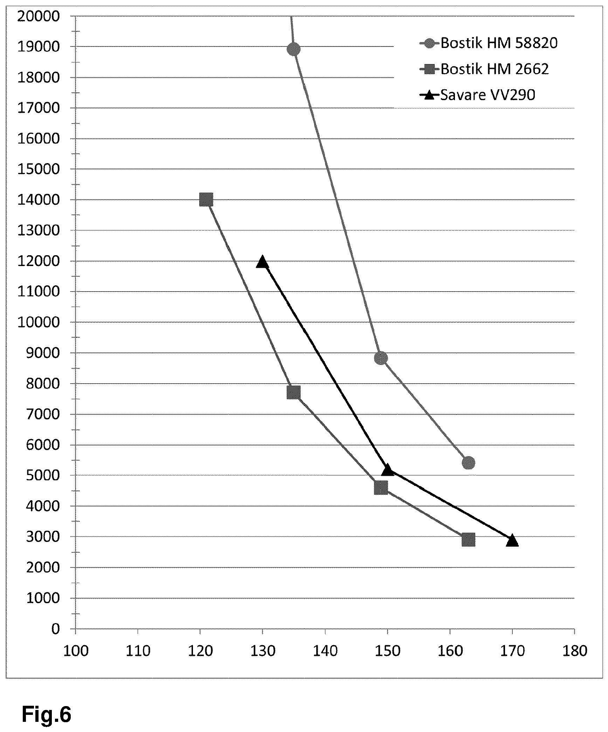

It is generally known to adapt or select viscosity of adhesives for process reasons, to ensure right melting and application temperatures along manufacturing lines. We have found that viscosity was also important to define appropriate adhesives for channeled absorbent articles during use. Without wishing to be bound by theory, although all hot melt adhesives show exponential values of viscosity at temperatures of usage of an absorbent article, and although most of hot melt adhesives show similar converging values of viscosity at 170° (see Fig.6), differences of behaviour in terms of viscosity in the range 130°C-160°C can be linked to the final behaviour of the adhesive, in particular its stability in wet state.

Preferably the adhesive of the present invention has a viscosity of at least 6000, more preferably at least 7000, even more preferably at least 7500, most preferably at least 8000 mPa·s at 150°C.

According to the present invention, the channel has a stability of preferably at least 20 minutes or at least 30 minutes, more preferably at least 45 minutes, even more preferably at least 1 hour, according to the Hang-Peel Test at 38°C as herein described.

We have also found that excellent channel stability could be reached with less or reasonable amount of adhesive, thereby avoiding high quantities of adhesives which otherwise can reduce the absorbent article flexibility and comfort and/or reduce absorption capacities and/or cost more. Advantageously the adhesive is present in the channel(s) in a quantity of at least 1 gsm, preferably at least 3 gsm, more preferably at least 4 gsm. It is advantageously present in a quantity of at most 10 gsm, preferably at most 7 gsm, more preferably at most 6 gsm. The adhesive is highly preferably present in the channel(s) in a quantity of between 4 and 6 gsm.

Preferably, the attachment zones comprise, preferably consist essentially of, more preferably consist of the hot melt adhesive according to the present invention. Advantageously, they are free of any other type of mechanical bonds like ultrasonic bonds or thermal bonds. Typically the upper and lower core wrap layers are, directly or indirectly, joined together at one or more positions inboard of the perimeter of the absorbent core and may be distinct to (or disconnected or inboardly spaced from) said perimeter; or come into contact with said perimeter. Preferably, the absorbent core is free of any channel extending up to the edges of the absorbent core.

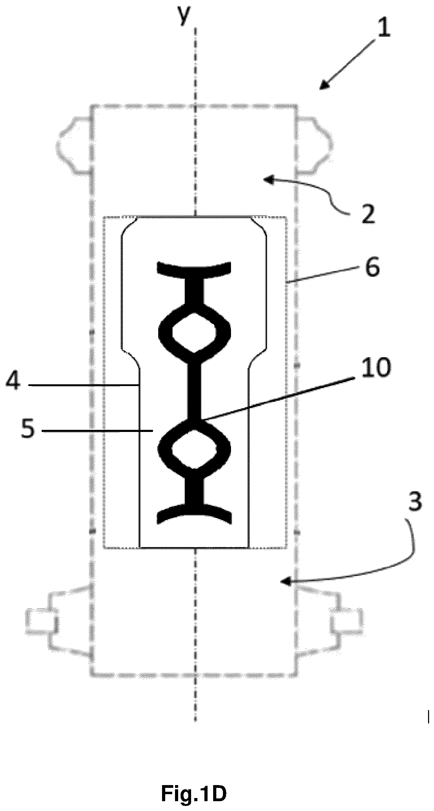

As exemplified in Fig. 1A-D, the top and bottom core wrap layers are joined together at one or more attachment zones to form one or more channels (10), preferably a single channel, like for example in Fig. 1A or 1D, substantially free of absorbent material.

In accordance with the invention, the channel(s) may be formed by straight lines and/or curved lines. Preferably, at least one channel follows a substantially continuous path from any point of said channel to any other point of the same channel. In a preferred single channel configuration, the channel is substantially U-shaped, like for example in Fig. 1A

In an embodiment, the top and bottom core wrap layers are adhered by one or more adhesives according to the invention. The adhesive may be either uniformly applied within the channels or it may be applied in zones of the channels such to form zones, preferably alternating zones, of different bonding strength between the core wrap layers. Ways to achieve stronger bonding strength in some zones may include using higher amounts of adhesive in said zones or applying greater mechanical pressure on said zones.

In an embodiment, the top and bottom core wrap layers are adhered along the channels, at a plurality of discrete joining areas, preferably wherein said discrete joining areas are free of adhesive and typically comprise mechanical bonds. Advantageously, this allows for a permanent bonding of the top and bottom layers of the core wrap that limits the presence of additional hydrophobic substances such as adhesives therebetween whilst maximizing the unbonded spaces therebetween to enhance the fluid flow through the channel.

In an embodiment the bonding strength in some zones of the channels is less than in others, and the top core wrap layer and bottom core wrap layer may separate in said zones. This arrangement may allow the bonding in some zones to fail in a controlled way upon for example swelling of the SAP such to allow more volume to be available for expansion thereof (and prevent early saturation or non-optimal absorption), with typically the zones resisting such expansion providing integrity of the channels even in wet state.

The core wrap layers are preferably nonwoven webs. Advantageously, at least one of the top and bottom core wrap layers is an elastic nonwoven (e.g. containing an elastic material such as Vistamaxx resin from ExxonMobil, or other suitable polymers capable of imparting elasticity to a nonwoven web). An advantage of this embodiment is that the nonwoven web better and more easily wraps around the 3D insert during the manufacturing process.

According to the invention, the absorbent core comprises absorbent material selected from the group consisting of cellulose fibers, superabsorbent polymers and combinations thereof. The absorbent material may in particular comprise cellulosic fluff and/or fibrous web typically comprising airlaid fibers of the cellulosic kind. The superabsorbent polymers may typically be in the form of a plurality of discrete particles that may be distributed within the absorbent material or directly agglomerated in one or more pockets between at least two nonwoven webs. The absorbent core may advantageously include cellulose fibers and superabsorbent polymers in a proportion of 5-55 Wt% cellulose and 45-95 Wt% SAP.

In an embodiment, the width of the channels is constant throughout a continuous path of a channel. Alternatively, it may vary along a channel. The width of the channels may be between 3 and 20 mm, preferably between 5 and 15 mm, more preferably between 5 and 12 mm.

The cores herein may have various shapes. Preferably the width of the core in the region of the transverse center line is less than in at least one of the front or back portions. This region may be positioned in a crotch portion of the absorbent article such to provide better ergonomics and fit along the leg of a wearer. It is preferred that said cores are symmetric at least about the longitudinal axis thereof. Irrespective of the core geometry, it is understood herein that the same or similar channels as described herein may be interchangeably used.

The absorbent articles may further comprise an acquisition distribution layer (ADL), also referred to herein as acquisition and distribution system, positioned between said topsheet and said core. The ADL may be positioned at a body-facing side of the absorbent core, between the topsheet and the absorbent core of the absorbent article, and more preferably in close proximity or even in good contact (most preferably in direct contact) with the body-facing side of the absorbent core. The use of an ADL in combination with the channels of the present invention may lead to an extremely good distribution of fluids from a discharge area to the entire absorbent core whilst attaining excellent perceived dryness performance.

The acquisition distribution system may be a single layer of spunbond and/or carded (e.g. carded thermobonded) nonwoven. Alternatively, the acquisition distribution system may be multi-layered and comprise at least one spunbond layer and at least one meltblown layer typically the layers being nonwoven layers. Preferably, the acquisition distribution system is a nonwoven selected from the group consisting of: SM, SMS, SMMS, and combinations thereof.

In an embodiment, the acquisition distribution layer comprises a plurality of layers, wherein at least one of said layers, preferably each of said layers, consists of spunbond, meltblown and/or carded (e.g. thermocarded) nonwoven and wherein at least the layer most distal from the body-facing side of the absorbent core consists of spunbond and/or carded nonwoven, preferably wherein both the layer most distal and the layer most proximal to the body-facing (also referred to herein as "skin-facing") side of the absorbent core consists of spunbond and/or carded nonwoven.

In an embodiment, the acquisition distribution layer for use herein comprises synthetic fibers which are comprised at a level of greater than 80%wt by weight of said acquisition distribution layer. The acquisition distribution layer may have a basis weight of from 5 to 50 g/m², 10 to 50 g/m², preferably from 15 to 40 g/m², more preferably from 18 to 35 g/m², even more preferably from 20 to 30 g/m², most preferably from 21 to 25 g/m².

In an embodiment, the acquisition distribution system is the top layer of the core wrap and the absorbent article is free of additional layers, such as an acquisition distribution layer, so that the top layer of the core wrap is in direct contact with the topsheet. The present invention is especially beneficial in such embodiments which do not enclose a discrete, separate acquisition distribution layer, where distribution is mainly ensured by the channel(s) themselves and where channel(s) stability during entire use is thus important. Said top layer of the core wrap may comprise a spunbond and/or carded, e.g. carded thermobonded, nonwoven layer comprising synthetic fibers, wherein preferably said synthetic fibers are comprised at a level of greater than 80%wt by weight of said layer, and/or wherein said top layer of the core wrap has a basis weight of from 5 to 50 g/m². Alternatively, said top layer of the core wrap may be multi-layered and comprise at least one spunbond layer and at least one meltblown layer typically the layers being nonwoven layers. Preferably, the multi-layered top layer of the core wrap acting as acquisition distribution system is a nonwoven selected from the group consisting of: SM, SMS, SMMS, and combinations thereof. Advantageously, by choosing core wraps as described, improved performance on rewet may be achieved even whilst eliminating the presence of further acquisition distribution layers, thus further introducing cost benefits.

We have found that fluid distribution, in embodiments of the present absorbent articles which comprise an ADL, may depend on the relative size and positioning of the ADL with respect to the fluid distribution structure, and in particular the channel(s), of the absorbent core. The acquisition distribution layer may be asymmetrically positioned over the absorbent core, offset at least along the longitudinal axis, preferably positioned such that it does not overlap a portion of the channel at a position proximal to the back of the core, more preferably wherein the acquisition distribution layer is positioned such that it does not overlap with an ending section of the channel. This arrangement has the advantage of raw material cost saving by ensuring the ADL is positioned where it is needed most. Moreover by ensuring a part of the channel remains exposed (particularly the ending section of the channel) speed of liquid flow through the channel from front to back is not compromised.

Absorbent articles according to the present invention include disposable diapers or diaper pants; disposable incontinence diapers or diaper pants; sanitary pads, sanitary napkins and panty liners. They comprise a core sandwiched between a liquid permeable topsheet and a liquid impermeable backsheet. The backsheet may comprise a print or graphic viewable from the garment facing side of said article that substantially matches the shape and/or contour of the channel(s). This has the advantage to further accentuate the visual perception of the presence of such channel and its location in the absorbent article.

The absorbent articles herein are preferably diapers or pants for babies or adults. In case of diapers preferred further components used in the art include: elastic ears or side panels for the fastening thereof, fastening systems comprising hook and loop and/or adhesive; leg cuffs; transversal front and back barriers or cuffs; acquisition distribution layers, wetness indicators etc. In case of pants preferred are 3-piece pants wherein front and back elasticized belts are joined via an absorbent insert comprising the topsheet, backsheet and absorbent core therebetween. The elasticized belts typically comprise a plurality of elastic strands or unitary elastic film. Further components in pants may be similar to those found in diapers such as leg cuffs; transversal front and back barriers or cuffs; acquisition distribution layers, wetness indicators etc.

In a further aspect, the present disclosure relates to a process of making an absorbent core as herein described, comprising the steps of:
i. providing a mold comprising a non-porous 3D insert therein, typically said insert having the inverse shape of the desired channel(s), wherein the mold is in fluid communication with an under-pressure source except for said insert;
ii. applying a first nonwoven web to said mold;
iii. applying an absorbent material over at least a portion of said nonwoven web;
iv. removing said absorbent material from areas of the nonwoven web corresponding to said insert, such as by the under-pressure source being arranged to provide a vacuum force forcing said absorbent material around the insert to substantially evacuate a surface of the nonwoven web in contact thereto from said absorbent material or by mechanical means such as use of a brush;
v. applying a second nonwoven web directly or indirectly over the absorbent material, or folding said first nonwoven web, such to sandwich said absorbent material between an upper and lower layers of said nonwoven web(s);
vi. applying a bonding step to form a laminate comprising said first nonwoven, said second nonwoven and said absorbent material therebetween;
vii. optionally removing said laminate from the mold to form an absorbent core comprising channel(s) having the inverse shape of said insert.

Further embodiments of such process are for example described in EP3342386 A1, in paragraphs [0148] to [0160] and with reference to Figures 15A and 15B.

In a preferred embodiment, the bonding step comprises applying an adhesive on a surface of the second nonwoven web and joining said web to said first nonwoven web and/or absorbent material, preferably the adhesive being applied in continuous or discontinuous spaced apart stripes aligned with said channels such that the resulting core laminate comprises adhesive rich and adhesive poor regions, wherein the adhesive rich regions are substantially located along said channels and the adhesive poor regions are located in areas of the core other than said channels. An advantage of this embodiment is to limit the risk of adhering absorbent material within the channels and to rather directly bond the topsheet and backsheet nonwoven together at these channel locations.

In an embodiment, the mold comprises a plurality of perforations or openings across its surface typically forming channels arranged to be in fluid (preferably air) communication with the under pressure source. Preferably, the 3D insert is positioned above and/or over said mold surface comprising a plurality of said perforations or openings and said 3D insert being free of said perforations or openings and consists of a solid component that is not in fluid communication with the under pressure source.

Preferably, the 3D insert has a cross-sectional shape selected from the group consisting of square, rectangular, oval, semi-circular, and combinations thereof.

Alternatively to the use of a 3D insert, a process of making an absorbent core as herein described, in particular including a channel having a more complex shape and including numerous various attachment zones, may comprise the steps of:
i. providing a mold comprising a non-porous pattern, typically said pattern having the shape of the desired channel(s), and porous depressions outside of this pattern and in fluid communication with an under-pressure source;
ii. applying a first nonwoven web to said mold;
iii. applying an absorbent material over at least a portion of said nonwoven web, the under-pressure source being arranged to provide a vacuum force forcing said absorbent material into the porous depressions;
iv. applying a second nonwoven web directly or indirectly over the absorbent material, or folding said first nonwoven web, such to sandwich said absorbent material between an upper and lower layers of said nonwoven web(s);
v. applying a bonding step to form a laminate comprising said first nonwoven, said second nonwoven and said absorbent material therebetween;
vi. optionally removing said laminate from the mold to form an absorbent core comprising channel(s) having the shape of the non-porous pattern.

More preferably, the channel(s) are formed substantially only by the vacuum force and no additional mechanical action such as embossing.

Adhesive may be applied to the respective substrates via one or more adhesive applicators that may be arranged to apply a spray and/or slot-coating of adhesive on respective substrates.

The molds described herein above may be contained within the circumference of a rotating drum apparatus, said drum apparatus typically comprising a plurality of said molds along its circumference. Said drum apparatus may be integrated within existing apparatuses for forming absorbent core laminates. An advantage of such a simple arrangement is that it allows for the formation of such novel absorbent cores in a simple and effective manner without considerable capital investment to substantially change major parts of existing core forming equipment.

### TEST METHODS

### Hang Peel Test at 38°C:

This method is used to determine the bonding strength of the adhesive within the channels between top and bottom core wrap of an absorbent article under specified force and heat.

### Procedure

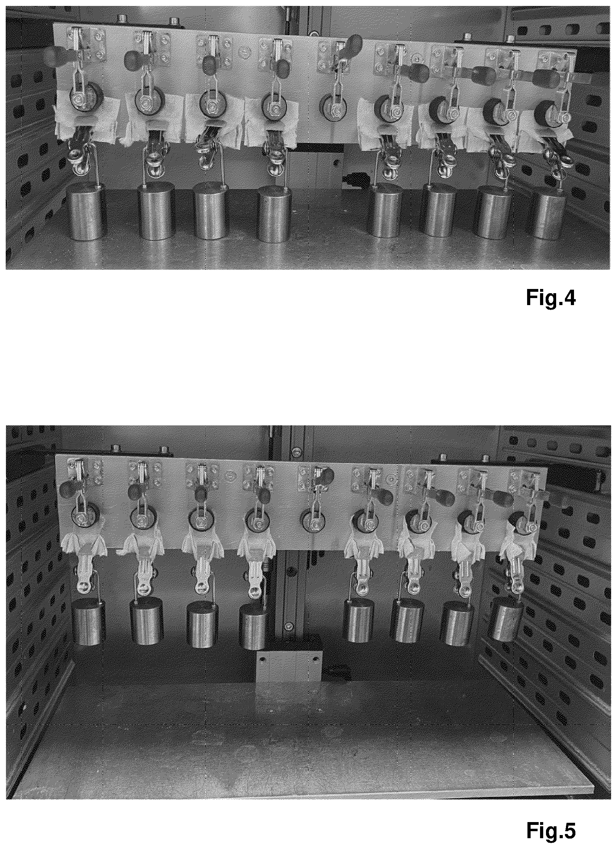

1. This test has to be performed on 4 unused absorbent articles.
2. Cut out with scissors, as illustrated in Fig. 2, a 4 cm x 5 cm sample (20) of the absorbent core (including top and bottom core wrap layers) centred on a channel (10), such that the 5 cm long side is parallel to the channel.
3. Carefully remove the absorbent material (5) without damaging the glueing in the channel (10), thereby freeing the core wrap layers on both side of the channels.
4. Heat the oven at a temperature of 38 °C.
5. Insert and position in the oven the samples with the channel horizontally placed, with one free core wrap layer (31) on one side of the channel between the upper jaws and add the 350g (±10 g) weights to the other free core wrap layer (32) of the same side of the channel, as shown in Fig. 3 and 4 (the hatched zone in Fig.3 represents the adhesive in the channel (10)). Do not release the platform carrying the weights, as shown in Fig. 4. Condition the samples and weights at 38°C for 15 minutes.
6. After 15 min, slowly release the platform as shown in Fig. 5, and start the stopwatch.
7. Note the times when the channel is broken in minutes : seconds.

An absorbent article is said to have a channel having a stability of at least 20, 30, 45, etc. minutes according to the Hang-Peel Test at 38°C as herein described, when at least three out of the four tests mentioned under point 1 of the procedure show a result of respectively at least 20, 30, 45, etc. minutes, i.e. are not broken before 20, 30, 45, etc. minutes, preferably when all four tests show a result of respectively at least 20, 30, 45, etc. minutes, i.e. none of the samples are broken before 20, 30, 45, etc. minutes.

### Viscosity measurements:

All viscosity measurements referred to herein are apparent viscosity measurements in accordance with ASTM D3236-88 (2004), using the following particulars: RVT, SC 4-27, 20 rpm.

### EXAMPLES

Four 4 cm x 5 cm samples of absorbent cores with channel have been cut out according to the Hang-Peel Test at 38°C as herein described.

In example 1 according to the present invention, a rubber-based adhesive commercially available from BOSTIK under reference HM 58820, having a viscosity curve with circles as shown in Fig. 6, i.e. a viscosity at 150°C of 8830 mPa·s, was used to bond together a top core wrap layer and a bottom core wrap layer to form one channel substantially free of absorbent material. In example 1a the adhesive was present in the channel in a quantity of 3 gsm; in example 1b, in a quantity of 5 gsm.

The results of the Hang-Peel Test at 38°C are given in Table I below. They show an excellent stability of at least 45 minutes with 5 gsm of adhesive, and a slightly less reproducible result with a quantity of 3 gsm, where sometimes the stability is around 20 or 30 minutes or at least 45 minutes, but sometimes weaker. This may be explained by a quantity of adhesive within the channel which was not homogeneous enough. According to the present invention, example 1a shows a channel stability of at least 20 minutes (3 out of the 4 samples show this), and example 1b is said to show a channel stability of at least 45 minutes.

In comparative example 1, not in accordance with the present invention, a polyolefin-based adhesive commercially available from SAVARE under reference VV290, having a viscosity curve with triangles as shown in Fig. 6, i.e. a viscosity at 150°C of 5200 mPa·s, was used to bond together a top core wrap layer and a bottom core wrap layer to form one channel substantially free of absorbent material. In comparative example 1a the adhesive was present in the channel in a quantity of 3 gsm; in comparative example 1b, in a quantity of 5 gsm; and in comparative example 1c, in a quantity of 10 gsm. None of the samples resisted to the Hang-Peel Test at 38°C more than 1 minute and 25 seconds.

In comparative example 2, not in accordance with the present invention, a polyolefin-based adhesive commercially available from BOSTIK under reference HM 2662, having a viscosity curve with squares as shown in Fig. 6, i.e. a viscosity at 150°C of 4600 mPa·s, was used to bond together a top core wrap layer and a bottom core wrap layer to form one channel substantially free of absorbent material. In comparative example 2a the adhesive was present in the channel in a quantity of 10 gsm. Despite such high amount of adhesive, none of the samples resisted to the Hang-Peel Test at 38°C more than 32 seconds.

| **TABLE I** | | sample 1 | sample 2 | sample 3 | sample 4 |
|---|---|---|---|---|---|
| Comp. Ex. 1a | SAVARE VV290 3gsm | 00:21 | 00:22 | 00:29 | 00:36 |
| Comp. Ex. 1b | SAVARE VV290 5gsm | 00:48 | 00:59 | 00:51 | 01:04 |
| Comp. Ex. 1c | SAVARE VV290 10gsm | 01:02 | 01:03 | 01:25 | 01:09 |
| Comp. Ex. 2a | Bostik HM 2662 10gsm | 00:12 | 00:08 | 00:16 | 00:32 |
| Example 1a | Bostik HM 58820 3gsm | 04:19 | ≥45:00 | 21:31 | 27:02 |
| Example 1b | Bostik HM 58820 5gsm | ≥45:00 | ≥45:00 | ≥45:00 | ≥45:00 |

## Claims

1. An absorbent article comprising a liquid permeable topsheet, a liquid impermeable backsheet and an absorbent core positioned between said topsheet and said backsheet, said absorbent core comprising absorbent material selected from the group consisting of cellulose fibers, superabsorbent polymers and combinations thereof, wherein said absorbent material is contained within at least one core wrap substrate enclosing said absorbent material, and wherein a top layer of said core wrap is bonded via a hot melt adhesive to a bottom layer of said core wrap at one or more attachment zones to form one or more channels substantially free of absorbent material, **characterized in that** said hot melt adhesive is selected from the group consisting of rubber-based adhesives, adhesives having a viscosity of at least 5500 mPa·s at 150°C and rubber-based adhesives having a viscosity of at least 5500 mPa·s at 150°C.

2. An absorbent article according to claim 1, wherein the adhesive has a viscosity of at least 6000 mPa·s at 150°C.

3. An absorbent article according to claim 1 or claim 2, wherein the channel has a stability of at least 20 minutes according to the Hang-Peel Test at 38°C as herein described.

4. An absorbent article according to any of the preceding claims, wherein the channel has a stability of at least 30 minutes according to the Hang-Peel Test at 38°C as herein described.

5. An absorbent article according to any of the preceding claims, wherein the attachment zones form a single channel.

6. An absorbent article according to any of the preceding claims, wherein the absorbent core is free of any channel extending up to the edges of the absorbent core.

7. An absorbent article according to any of the preceding claims, wherein the adhesive is present in the channel(s) in a quantity of at least 3 gsm.

8. An absorbent article according to any of the preceding claims, wherein the adhesive is present in the channel(s) in a quantity of at least 4 gsm.

9. An absorbent article according to any of the preceding claims, wherein an acquisition distribution system is present between the topsheet and the absorbent core, said system being a single layer of spunbond and/or carded nonwoven.

10. An absorbent article according to any of the claims 1 to 8, wherein an acquisition distribution system is present between the topsheet and the absorbent core, said system being multi-layered and comprising at least one spunbond layer and at least one meltblown layer.

11. An absorbent article according to any of the claims 1 to 8, wherein the top layer of the core wrap is in direct contact with the topsheet and comprises a spunbond and/or carded nonwoven layer.

12. An absorbent article according to any of the claims 1 to 8, wherein the top layer of the core wrap is in direct contact with the topsheet, is multi-layered and comprises at least one spunbond layer and at least one meltblown layer.

## Patentansprüche

1. Absorbierender Artikel, umfassend eine flüssigkeitsdurchlässige Oberschicht, eine flüssigkeitsundurchlässige Unterschicht und einen absorbierenden Kern, der zwischen der Oberschicht und der Unterschicht angeordnet ist, wobei der absorbierende Kern absorbierendes Material umfasst, das aus der Gruppe ausgewählt ist, bestehend aus Zellulosefasern, superabsorbierenden Polymeren und Kombinationen davon, wobei das absorbierende Material in mindestens einem Kernumwicklungssubstrat enthalten ist, das das absorbierende Material umschließt, und wobei eine obere Schicht der Kernumwicklung über einen Heißschmelzklebstoff an einer oder mehreren Befestigungszonen an eine untere Schicht der Kernumwicklung gebunden ist, um einen oder mehrere Kanäle zu bilden, die im Wesentlichen frei von absorbierendem Material sind, **dadurch gekennzeichnet, dass** der Heißschmelzklebstoff aus der Gruppe ausgewählt ist, bestehend aus Klebstoffen auf Kautschukbasis, Klebstoffen mit einer Viskosität von mindestens 5500 mPa·s bei 150 °C und Klebstoffen auf Kautschukbasis mit einer Viskosität von mindestens 5500 mPa·s bei 150 °C.

2. Absorbierender Artikel nach Anspruch 1, wobei der Klebstoff eine Viskosität von mindestens 6000 mPa·s bei 150 °C aufweist.

3. Absorbierender Artikel nach Anspruch 1 oder Anspruch 2, wobei der Kanal gemäß der hierin beschriebenen Hang-Peel-Prüfung bei 38 °C eine Stabilität von mindestens 20 Minuten aufweist.

4. Absorbierender Artikel nach einem der vorstehenden Ansprüche, wobei der Kanal gemäß der hierin beschriebenen Hang-Peel-Prüfung bei 38 °C eine Stabilität von mindestens 30 Minuten aufweist.

5. Absorbierender Artikel nach einem der vorstehenden Ansprüche, wobei die Befestigungszonen einen einzelnen Kanal bilden.

6. Absorbierender Artikel nach einem der vorstehenden Ansprüche, wobei der absorbierende Kern frei von jeglichen Kanälen ist, die sich bis zu den Rändern des absorbierenden Kerns erstrecken.

7. Absorbierender Artikel nach einem der vorstehenden Ansprüche, wobei der Klebstoff in dem Kanal/den Kanälen in einer Menge von mindestens 3 g/m² vorhanden ist.

8. Absorbierender Artikel nach einem der vorstehenden Ansprüche, wobei der Klebstoff in dem Kanal/den Kanälen in einer Menge von mindestens 4 g/m² vorhanden ist.

9. Absorbierender Artikel nach einem der vorstehenden Ansprüche, wobei zwischen dem zwischen der Oberschicht und dem absorbierenden Kern ein Aufnahmeverteilungssystem vorhanden ist, wobei das System eine einzelne Schicht aus Spinnvlies und/oder kardiertem Vliesstoff ist.

10. Absorbierender Artikel nach einem der Ansprüche 1 bis 8, wobei zwischen der Oberschicht und dem absorbierenden Kern ein Aufnahmeverteilungssystem vorhanden ist, wobei das System mehrschichtig ist und mindestens eine Spinnvliesschicht und mindestens eine Meltblown-Schicht umfasst.

11. Absorbierender Artikel nach einem der Ansprüche 1 bis 8, wobei die obere Schicht der Kernumwicklung in direktem Kontakt mit der Oberschicht steht und eine Spinnvlies- und/oder kardierte Vliesschicht umfasst.

12. Absorbierender Artikel nach einem der Ansprüche 1 bis 8, wobei die obere Schicht der Kernumwicklung in direktem Kontakt mit der Oberschicht steht, mehrschichtig ist und mindestens eine Spinnvliesschicht und mindestens eine Meltblown-Schicht umfasst.

## Revendications

1. Article absorbant comprenant une feuille supérieure perméable aux liquides, une feuille arrière imperméable aux liquides et une âme absorbante placée entre ladite feuille supérieure et ladite feuille arrière, ladite âme absorbante comprenant un matériau absorbant choisi dans le groupe constitué de fibres de cellulose, polymères superabsorbants et de combinaisons de ceux-ci, dans lequel ledit matériau absorbant est contenu à l'intérieur d'au moins un substrat d'enveloppe d'âme renfermant ledit matériau absorbant, et dans lequel une couche supérieure de ladite enveloppe d'âme est liée, par l'intermédiaire d'un adhésif thermofusible, à une couche inférieure de ladite enveloppe d'âme au niveau d'une ou plusieurs zones de fixation pour former un ou plusieurs canaux sensiblement exempts de matériau absorbant, **caractérisé en ce que** ledit adhésif thermofusible est choisi dans le groupe constitué d'adhésifs à base de caoutchouc, adhésifs ayant une viscosité d'au moins 5 500 mPa·s à 150 °C et adhésifs à base de caoutchouc ayant une viscosité d'au moins 5 500 mPa·s à 150 °C.

2. Article absorbant selon la revendication 1, dans lequel l'adhésif a une viscosité d'au moins 6 000 mPa·s à 150 °C.

3. Article absorbant selon la revendication 1 ou la revendication 2, dans lequel le canal a une stabilité d'au moins 20 minutes selon le test de suspension et de pelage à 38 °C tel que décrit ici.

4. Article absorbant selon l'une quelconque des revendications précédentes, dans lequel le canal a une stabilité d'au moins 30 minutes selon le test de suspension et de pelage à 38 °C tel que décrit ici.

5. Article absorbant selon l'une quelconque des revendications précédentes, dans lequel les zones de fixation forment un canal unique.

6. Article absorbant selon l'une quelconque des revendications précédentes, dans lequel l'âme absorbante est exempte de tout canal s'étendant jusqu'aux bords de l'âme absorbante.

7. Article absorbant selon l'une quelconque des revendications précédentes, dans lequel l'adhésif est présent dans le ou les canaux en une quantité d'au moins 3 g/m².

8. Article absorbant selon l'une quelconque des revendications précédentes, dans lequel l'adhésif est présent dans le ou les canaux en une quantité d'au moins 4 g/m².

9. Article absorbant selon l'une quelconque des revendications précédentes, dans lequel un système de récupération répartition est présent entre la feuille supérieure et l'âme absorbante, ledit système étant une monocouche de non-tissé filé-lié et/ou cardé.

10. Article absorbant selon l'une quelconque des revendications 1 à 8, dans lequel un système de récupération répartition est présent entre la feuille supérieure et l'âme absorbante, ledit système étant multicouche et comprenant au moins une couche filée-liée et au moins une couche extrudée-soufflée.

11. Article absorbant selon l'une quelconque des revendications 1 à 8, dans lequel la couche supérieure de l'enveloppe d'âme est en contact direct avec la feuille supérieure et comprend une couche de non-tissé filé-lié et/ou cardé.

12. Article absorbant selon l'une quelconque des revendications 1 à 8, dans lequel la couche supérieure de l'enveloppe d'âme est en contact direct avec la feuille supérieure, est multicouche et comprend au moins une couche filée-liée et au moins une couche extrudée-soufflée.
